# FASCICULE DE BREVET EUROPEEN

(11) **EP 2 890 390 B1**
(45) Date de publication et mention de la délivrance du brevet: **16.11.2016**
(21) Numéro de dépôt: 13774714.3
(22) Date de dépôt: 29.08.2013
(51) Int. Cl.: A61K 38/26, A61K 38/22, A61P 19/02, A61K 45/06

(54) **TRAITEMENT DE L'ARTHROSE PAR LES HORMONES INCRÉTINES OU LEURS ANALOGUES**
BEHANDLUNG VON OSTEOARTHRITIS MIT INKRETINHORMONEN ODER ANALOGA DAVON
TREATMENT OF OSTEOARTHRITIS WITH INCRETIN HORMONES OR ANALOGUES THEREOF

(30) Priorité: 30.08.2012 FR 1258100
(43) Date de publication de la demande: 08.07.2015
(73) Titulaire: Université Pierre et Marie Curie (Paris 6), 75005 Paris (FR); Assistance Publique - Hôpitaux de Paris, 75004 Paris (FR)
(72) Inventeur: BERENBAUM, Francis, F-91190 Gif Sur Yvette (FR); BOUGAULT, Carole, F-69009 Lyon (FR); ATTALI, Claire, F-92160 Antony (FR)
(74) Mandataire: Pierru, Bénédicte
(86) Numéro de dépôt international: PCT/FR2013/051998
(87) Numéro de publication internationale: WO 2014/023923

(56) Documents cités:
- DICEMBRINI I ET AL: "Bone: Incretin hormones perceiver or receiver?", EXPERIMENTAL DIABETES RESEARCH 2012 HINDAWI PUBLISHING CORPORATION USA, vol. 2012, 17 juin 2012 (2012-06-17), XP002692445, ISSN: 1687-5214
- DING KE-HONG ET AL: "Impact of glucose-dependent insulinotropic peptide on age-induced bone loss", JOURNAL OF BONE AND MINERAL RESEARCH, vol. 23, no. 4, avril 2008 (2008-04), pages 536-543, XP002692446, ISSN: 0884-0431
- VITTONE F ET AL: "Sitagliptin reduces plaque macrophage content and stabilises arteriosclerotic lesions in Apoe (-/-) mice", DIABETOLOGIA, vol. 55, no. 8, 18 mai 2012 (2012-05-18), pages 2267-2275, XP002692447,
- CHEN TIEN-HSING ET AL: "Exendin-4 attenuates lipopolysaccharides induced inflammatory response but does not protects H9c2 cells from apoptosis", IMMUNOPHARMACOLOGY AND IMMUNOTOXICOLOGY, vol. 34, no. 3, juin 2012 (2012-06), pages 484-490, XP009167236,

### SEQUENCE LISTING

<110> Université Pierre et Marie Curie (PARIS 6)
<120> Traitement de l'arthrose par les hormones incrétines ou leurs
   analogues
<130> B1384PC
<160> 16
<170> PatentIn version 3.3
<210> 1
   <211> 180
   <212> PRT
   <213> Homo sapiens
<400> 1
   Met Lys Ser Ile Tyr Phe Val Ala Gly Leu Phe Val Met Leu Val Gln
   1               5                   10                  15
   Gly Ser Trp Gln Arg Ser Leu Gln Asp Thr Glu Glu Lys Ser Arg Ser
               20                  25                  30
   Phe Ser Ala Ser Gln Ala Asp Pro Leu Ser Asp Pro Asp Gln Met Asn
           35                  40                  45
   Glu Asp Lys Arg His Ser Gln Gly Thr Phe Thr Ser Asp Tyr Ser Lys
       50                  55                  60
   Tyr Leu Asp Ser Arg Arg Ala Gln Asp Phe Val Gln Trp Leu Met Asn
   65                  70                  75                  80
   Thr Lys Arg Asn Arg Asn Asn Ile Ala Lys Arg His Asp Glu Phe Glu
                   85                  90                  95
   Arg His Ala Glu Gly Thr Phe Thr Ser Asp Val Ser Ser Tyr Leu Glu
               100                 105                 110
   Gly Gln Ala Ala Lys Glu Phe Ile Ala Trp Leu Val Lys Gly Arg Gly
           115                 120                 125
   Arg Arg Asp Phe Pro Glu Glu Val Ala Ile Val Glu Glu Leu Gly Arg
       130                 135                 140
   Arg His Ala Asp Gly Ser Phe Ser Asp Glu Met Asn Thr Ile Leu Asp
   145                 150                 155                 160
   Asn Leu Ala Ala Arg Asp Phe Ile Asn Trp Leu Ile Gln Thr Lys Ile
                   165                 170                 175
   Thr Asp Arg Lys
               180
<210> 2
   <211> 37
   <212> PRT
   <213> Homo sapiens
<400> 2
   His Asp Glu Phe Glu Arg His Ala Glu Gly Thr Phe Thr Ser Asp Val
   1               5                   10                  15
   Ser Ser Tyr Leu Glu Gly Gln Ala Ala Lys Glu Phe Ile Ala Trp Leu
               20                  25                  30
   Val Lys Gly Arg Gly
           35
<210> 3
   <211> 31
   <212> PRT
   <213> Homo sapiens
<400> 3
   His Ala Glu Gly Thr Phe Thr Ser Asp Val Ser Ser Tyr Leu Glu Gly
   1               5                   10                  15
   Gln Ala Ala Lys Glu Phe Ile Ala Trp Leu Val Lys Gly Arg Gly
               20                  25                  30
<210> 4
   <211> 30
   <212> PRT
   <213> Homo sapiens
<400> 4
   His Ala Glu Gly Thr Phe Thr Ser Asp Val Ser Ser Tyr Leu Glu Gly
   1               5                   10                  15
   Gln Ala Ala Lys Glu Phe Ile Ala Trp Leu Val Lys Gly Arg
               20                  25                  30
<210> 5
   <211> 153
   <212> PRT
   <213> Homo sapiens
<400> 5
   Met Val Ala Thr Lys Thr Phe Ala Leu Leu Leu Leu Ser Leu Phe Leu
   1               5                   10                  15
   Ala Val Gly Leu Gly Glu Lys Lys Glu Gly His Phe Ser Ala Leu Pro
   20 25 30
   Ser Leu Pro Val Gly Ser His Ala Lys Val Ser Ser Pro Gln Pro Arg
   35 40 45
   Gly Pro Arg Tyr Ala Glu Gly Thr Phe Ile Ser Asp Tyr Ser Ile Ala
   50 55 60
   Met Asp Lys Ile His Gln Gln Asp Phe Val Asn Trp Leu Leu Ala Gln
   65 70 75 80
   Lys Gly Lys Lys Asn Asp Trp Lys His Asn Ile Thr Gln Arg Glu Ala
   85 90 95
   Arg Ala Leu Glu Leu Ala Ser Gln Ala Asn Arg Lys Glu Glu Glu Ala
   100 105 110
   Val Glu Pro Gln Ser Ser Pro Ala Lys Asn Pro Ser Asp Glu Asp Leu
   115 120 125
   Leu Arg Asp Leu Leu Ile Gln Glu Leu Leu Ala Cys Leu Leu Asp Gln
   130 135 140
   Thr Asn Leu Cys Arg Leu Arg Ser Arg
   145 150
<210> 6
   <211> 42
   <212> PRT
   <213> Homo sapiens
<400> 6
   Tyr Ala Glu Gly Thr Phe Ile Ser Asp Tyr Ser Ile Ala Met Asp Lys
   1 5 10 15
   Ile His Gln Gln Asp Phe Val Asn Trp Leu Leu Ala Gln Lys Gly Lys
   20 25 30
   Lys Asn Asp Trp Lys His Asn Ile Thr Gln
   35 40
<210> 7
   <211> 23
   <212> DNA
   <213> Artificial
<220>
   <223> amorce antisens HPRT
<400> 7
   attcaaatcc ctgaagtact cat 23
<210> 8
   <211> 17
   <212> DNA
   <213> Artificial
<220>
   <223> amorce sens HPRT
<400> 8
   aggacctctc gaagtgt 17
<210> 9
   <211> 20
   <212> DNA
   <213> Artificial
<220>
   <223> amorce antisens GLP1R
<400> 9
   cagtcggcag cctagagagt 20
<210> 10
   <211> 18
   <212> DNA
   <213> Artificial
<220>
   <223> amorce sens GLP1R
<400> 10
   ctgcccagca acaccagt 18
<210> 11
   <211> 21
   <212> DNA
   <213> Artificial
<220>
   <223> amorce sens MMP-3
<400> 11
   tgaaaatgaa gggtcttccg g 21
<210> 12
   <211> 20
   <212> DNA
   <213> Artificial
<220>
   <223> amorce antisens MMP-3
<400> 12
   gcagaagctc cataccagca 20
<210> 13
   <211> 19
   <212> DNA
   <213> Artificial
<220>
   <223> amorce sens MMP-9
<400> 13
   aactacggtc gcgtccact 19
<210> 14
   <211> 20
   <212> DNA
   <213> Artificial
<220>
   <223> amorce antisens MMP-9
<400> 14
   ccacagccaa ctatgaccag 20
<210> 15
   <211> 21
   <212> DNA
   <213> Artificial
<220>
   <223> amorce sens MMP-13
<400> 15
   tgatggcact gctgacatca t 21
<210> 16
   <211> 20
   <212> DNA
   <213> Artificial
<220>
   <223> amorce antisens MMP-13
<400> 16
   tgtagccttt ggaactgctt 20

## Description

La présente invention relève du domaine de la médecine, et plus particulièrement de celui du traitement de l'arthrose.

### ARRIERE-PLAN TECHNOLOGIQUE DE L'INVENTION

L'arthrose ou arthropathie chronique dégénérative est une maladie articulaire chronique caractérisée par une détérioration structurale du cartilage articulaire. Les symptômes de cette pathologie peuvent varier selon l'articulation concernée mais sont en général caractérisés par une douleur persistante associée à une gêne fonctionnelle, c'est-à-dire à une limitation de la mobilité de l'articulation concernée.

Le cartilage articulaire est un tissu conjonctif composé de chondrocytes et d'une matrice extracellulaire essentiellement formée d'eau, de protéoglycanes et de collagène. Les chondrocytes ont un rôle fondamental dans l'homéostasie de la matrice extracellulaire dont ils assurent la synthèse et le renouvellement. Le maintien du cartilage dépend donc d'échanges complexes continus entre les chondrocytes et la matrice et est soumis en permanence à un équilibre critique entre les mécanismes de dégradation, sous l'influence de cytokines destructrices notamment les cytokines pro-inflammatoires TNF-α et IL-1β, et les mécanismes de synthèse ou de restauration sous l'effet de cytokines modulatrices et des facteurs de croissance, notamment IGF-1, TGF-β, et certaines BMP (« Bone morphogenetic proteins »).

La destruction arthrosique du cartilage est le résultat d'un déséquilibre entre les mécanismes d'anabolisme et de catabolisme de la matrice extracellulaire. Plusieurs facteurs peuvent favoriser la rupture de l'homéostasie de la matrice notamment des facteurs mécaniques liés par exemple à une surcharge articulaire chez un patient obèse, un traumatisme, des microtraumatismes répétés ou des vices architecturaux de l'articulation, des facteurs métaboliques, génétiques, hormonaux, ou encore le vieillissement. Cependant, l'initiation du processus arthrosique reste encore à ce jour très mal comprise.

Le déséquilibre entre les mécanismes d'anabolisme et de catabolisme se traduit essentiellement par une augmentation de la synthèse des métalloprotéases (MMP) (Blanc et al., 1999), une diminution de la synthèse des TIMP (« tissue inhibitors of metalloproteinases », les inhibiteurs physiologiques des MMP) et une inhibition de la synthèse des constituants de la matrice par les chondrocytes. Ce déséquilibre est accentué par un phénomène d'apoptose chondrocytaire accélérée (Hashimoto et al., 1998) et par l'activation chondrocytaire via différents médiateurs libérés par le tissu synovial (Sellam and Berenbaum, 2010). La cytokine pro-inflammatoire IL-1β synthétisée par les chondrocytes et les synoviocytes, a un rôle majeur dans ce processus de destruction arthrosique. Elle induit non seulement l'augmentation de la production de MMP par les chondrocytes mais également la réduction des capacités anaboliques et l'apoptose de ces cellules (Goldring et al., 2008). De plus, l'os sous-chondral participe aussi aux phénomènes dégradatifs de la matrice notamment par le biais de la sécrétion d'enzymes protéolytiques par les ostéoblastes (Sanchez et al., 2012).

Les métalloprotéases matricielles (MMP) impliquées dans la protéolyse matricielle de l'arthrose sont des collagénases, des stromélysines, des gélatinases et des métalloprotéases membranaires (Rannou et al., 2005). Toutes ces enzymes ne sont pas spécifiques du cartilage et sont impliquées dans de nombreux processus physiologiques notamment dans le remodelage de nombreux tissus conjonctifs (Nagase et al., 1999). Les collagénases interstitielles (MMP-1,-8,-13) sont capables de dégrader les collagènes I, II, III, IV, et VII. Le collagène ainsi dénaturé par ces enzymes devient un substrat pour les gélatinases. Les stromélysines (MMP-3,-10,-11) sont capables de dégrader les protéoglycanes, la gélatine, la fibronectine et le collagène de type IX mais seule MMP-3 semble impliquée dans la dégradation de la matrice cartilagineuse (Stove et al., 2001). Les gélatinases (MMP-2, et -9) dégradent le collagène interstitiel dénaturé et les collagènes de type IV et V. En outre, il a été démontré que les MMP-1,-3, et-13 sont également capables de dégrader les protéoglycanes (Little et al., 2002). D'autres enzymes, en particulier les aggrécanases ADAMTS-4 et -5, joueraient également un rôle dans la protéolyse matricielle (Fosang and Little, 2008).

En plus des métalloprotéases, d'autres médiateurs cataboliques participent à la dégénérescence arthrosique, notamment la prostaglandine E2 (PGE2) qui est impliquée dans la dégradation du cartilage et l'apoptose des chondrocytes (Hardy et al., 2002 ; Miwa et al., 2000).

Les traitements proposés aux patients atteints d'arthrose sont symptomatiques car, à l'heure actuelle, il n'existe pas de traitement curatif de cette pathologie. Les traitements médicamenteux sont des traitements symptomatiques à action immédiate (antalgiques, anti-inflammatoires non stéroïdiens) ou à action retardée (par exemple des médicaments comprenant du sulfate de chondroïtine (Structum, Chondrosulf), la diacereine (Art 50, Zondar), des extraits insaponifiables d'avocat et de soja (Piasclédine) ou de l'acide hyaluronique.

Du fait de leur rôle clé dans la destruction du cartilage, les métalloprotéases sont devenues des cibles privilégiées dans la recherche de nouveaux composés capables de ralentir ou stopper l'évolution de l'arthrose. Cependant, ces protéines sont impliquées dans de nombreux processus physiologiques et leur inhibition peut produire des effets secondaires imprévisibles. Cela fut, par exemple, le cas avec le composé PG-116800 dont la toxicité musculo-squelettique a été révélée lors d'essais cliniques (Krzeski et al., 2007).

Actuellement, l'arthrose concernerait environ neuf à dix millions de personnes en France dont 4,6 millions avec une arthrose symptomatique. Compte tenu du vieillissement de la population ainsi que de l'augmentation de la prévalence de l'obésité dans les pays développés, une très forte augmentation du nombre de patients arthrosiques est attendue dans les années à venir. Cette augmentation sera extrêmement coûteuse à la fois en terme de qualité de vie mais également du point de vue économique pour la prise en charge des patients. Il demeure donc crucial de développer rapidement de nouvelles stratégies pour inhiber ou ralentir la destruction du cartilage chez le sujet arthrosique.

### RESUME DE L'INVENTION

L'objectif de la présente invention est de fournir de nouveaux composés pouvant être utilisés dans le traitement de l'arthrose.

Les inventeurs ont démontré que les hormones incrétines GLP-1 (« Glucagon-like peptide 1 ») et GIP (« Gastric inhibitory peptide » ou « glucose-dependent insulinotropic polypeptide ») sont capables d'inhiber la surexpression de plusieurs métalloprotéases (MMP-3, MMP-13 et MMP-9) et de la prostaglandine E2 (PGE2) dans les ostéoblastes et les chondrocytes en réponse à la cytokine pro-inflammatoire IL-1β. Ils ont ainsi montré que les hormones incrétines sont capables de bloquer la surexpression de plusieurs médiateurs prodégradatifs impliqués dans le processus de destruction arthrosique.

Ainsi, la présente invention concerne une hormone incrétine ou un analogue de celle-ci, pour une utilisation dans le traitement de l'arthrose. Plus particulièrement, l'invention concerne un peptide choisi dans le groupe constitué du peptide GLP-1, du peptide GIP et des analogues de ceux-ci résistant à la dipeptidyl-peptidase IV (DPP-IV), pour une utilisation dans le traitement de l'arthrose.

Le peptide utilisé selon l'invention peut notamment comprendre une séquence choisie dans le groupe constitué des séquences SEQ ID NO: 1 à 6.

En particulier, le peptide peut être choisi dans le groupe constitué du peptide GLP-1 (7-36)- amide (SEQ ID NO: 4), du peptide GLP-1 (7-37) (SEQ ID NO: 3) et du peptide GIP (1-42) (SEQ ID NO: 6). De préférence le peptide est choisi dans le groupe constitué du peptide GLP-1 (7-36)- amide (SEQ ID NO: 4) et du peptide GLP-1 (7-37) (SEQ ID NO: 3).

Le peptide peut également être un analogue du peptide GLP-1 ou GIP résistant à la DPP-IV. De préférence, le peptide est un analogue du peptide GLP-1 résistant à la DPP-IV, en particulier un analogue choisi dans le groupe constitué de l'exenatide, le liraglutide, l'exendine-4, l'albiglutide, le taspoglutide, le lixisenatide, le LY315902, le dulaglutide (LY2189265), LY2199265, le LY2428757, le semaglutide (NN9535), le CJC-1131, le CJC-1134 et le ZP10. Le peptide peut également être un analogue choisi dans le groupe constitué de l'exenatide, le liraglutide, l'exendine-4, l'albiglutide, le taspoglutide, le lixisenatide, le LY315902, LY2199265, le LY2428757, le NN9535, le CJC-1131, le CJC-1134 et le ZP10. De manière tout particulièrement préférée, le peptide est un analogue choisi dans le groupe constitué de l'exenatide et du liraglutide.

Le peptide utilisé selon l'invention peut être utilisé dans le traitement de l'arthrose en combinaison avec une ou plusieurs autres substances actives. Dans ce cas, le peptide et la ou les autres substances actives sont administrés simultanément ou séquentiellement.

Le peptide utilisé selon l'invention peut notamment être utilisé en combinaison avec des inhibiteurs de l'enzyme dipeptidyl-peptidase IV, de préférences choisis dans le groupe constitué de sitagliptine, la saxagliptine, la vildagliptine, l'alogliptine et la linagliptine, ou d'autres substances telles que des antalgiques, des anti-inflammatoires non stéroïdiens, des anti-inflammatoires stéroïdiens et des antiarthrosiques à action lente. Le peptide peut également être administré en association avec des traitements locaux de l'arthrose.

Le peptide utilisé selon l'invention peut être administré sous sa forme mature, sous la forme d'un précurseur ou sous la forme d'un acide nucléique codant pour ledit peptide.

De préférence, le peptide utilisé selon l'invention est destiné à être administré par voie orale, sous-cutanée, intraveineuse ou intra-articulaire.

La présente invention concerne également une composition pharmaceutique comprenant un ou plusieurs peptides tels qu'utilisé selon l'invention, et un ou plusieurs supports et/ou excipients pharmaceutiquement acceptables, pour une utilisation dans le traitement de l'arthrose.

La présente invention concerne également une composition pharmaceutique comprenant un ou plusieurs peptides tels qu'utilisé selon l'invention, ou un ou plusieurs acides nucléiques codant pour un ou plusieurs peptides tels qu'utilisé selon l'invention, et un ou plusieurs supports et/ou excipients pharmaceutiquement acceptables, pour une utilisation dans le traitement de l'arthrose.

La composition peut en outre comprendre une ou plusieurs autres substances actives, notamment un ou plusieurs inhibiteurs de l'enzyme dipeptidyl-peptidase IV, de préférence choisis dans le groupe constitué de sitagliptine, la saxagliptine, la vildagliptine, l'alogliptine et la linagliptine, ou d'autres substances actives telles que des antalgiques, des anti-inflammatoires non stéroïdiens, des anti-inflammatoires stéroïdiens et des antiarthrosiques à action lente.

La composition peut être formulée sous la forme d'une composition ingérable ou injectable, de préférence sous la forme d'une composition destinée à être administré par voie orale, sous-cutanée, intraveineuse ou intra-articulaire.

### BREVE DESCRIPTION DES DESSINS

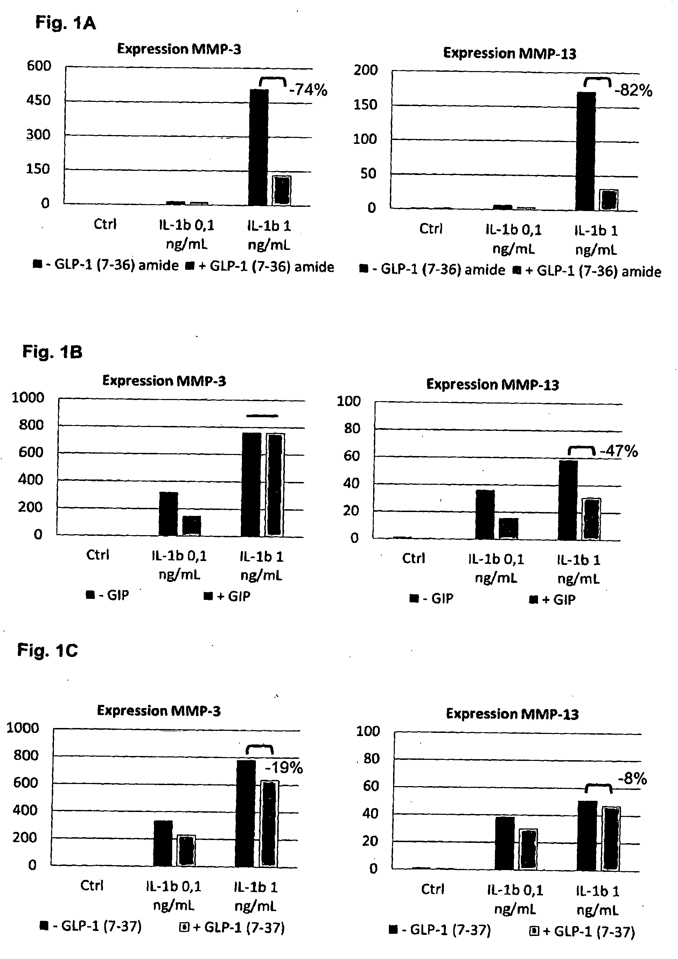

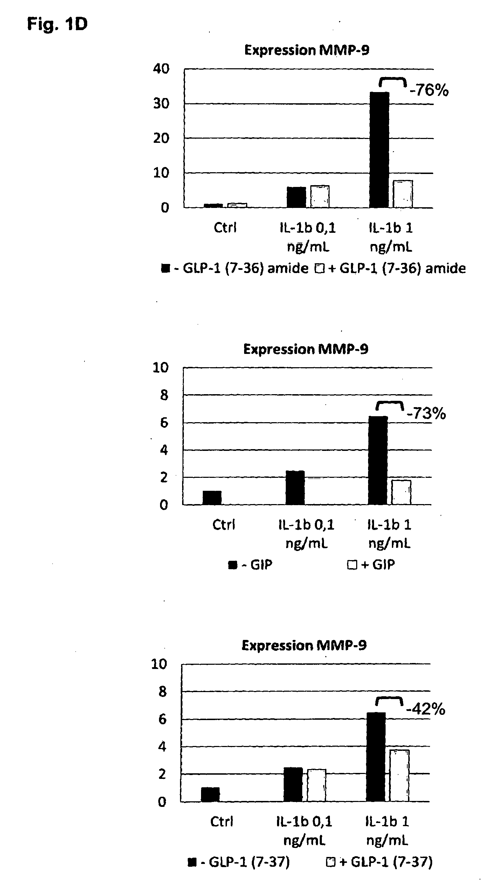

Figure 1 : Effet du peptide GLP-1 (7-36) amide, GLP-1 (7-37) ou GIP sur l'induction de l'expression des enzymes MMP-3, MMP-9 et MMP-13 dans les chondrocytes en réponse à IL-1β (0,1 ng/mL ou 1 ng/mL). Les valeurs obtenues par RT-PCR quantitative en temps réel sont normalisées par rapport aux valeurs obtenues avec le contrôle (sans IL-1β et sans GLP-1 ou GIP). A : expression de MMP-3 et MMP-13 en présence ou en absence de GLP-1 (7-36) amide (10 nM) ; B : expression de MMP-3 et MMP-13 en présence ou en absence de GIP (10 nM) ; C : expression de MMP-3 et MMP-13 en présence ou en absence de GLP-1 (7-37) (10 nM); D: expression de MMP-9 en présence ou en absence de GLP-1 (7-36) amide (10 nM), de GLP-1 (7-37) (10 nM) ou de GIP (10 nM).

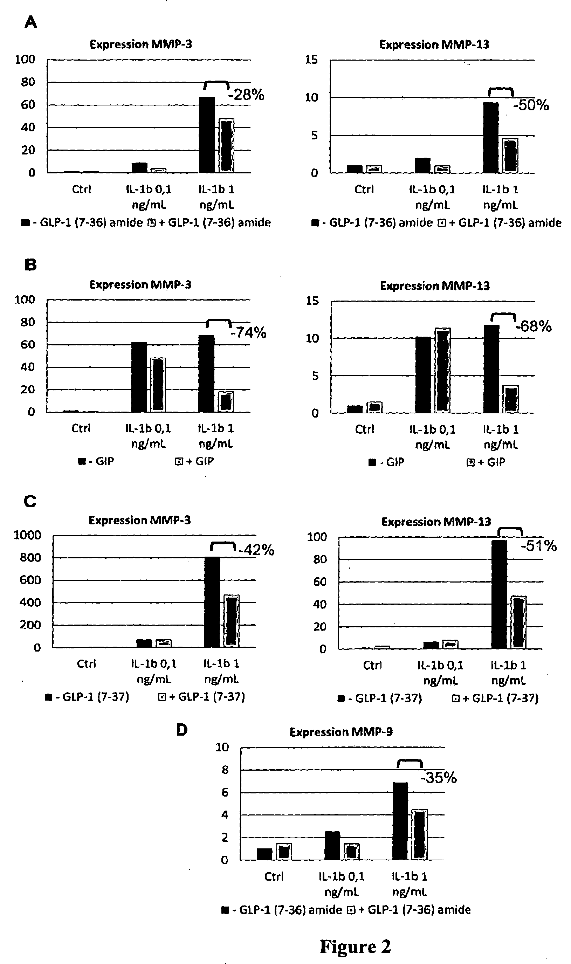

Figure 2 : Effet du peptide GLP-1 (7-36) amide, GLP-1 (7-37) ou GIP sur l'induction de l'expression des enzymes MMP-3, MMP-9 et MMP-13 dans les ostéoblastes en réponse IL-1β (0,1 ng/mL ou 1 ng/mL). Les valeurs obtenues par RT-PCR quantitative en temps réel sont normalisées par rapport aux valeurs obtenues avec le contrôle (sans IL-1β et sans GLP-1 ou GIP). A : expression de MMP-3 et MMP-13 en présence ou en absence de GLP-1 (7-36) amide (10 nM) ; B : expression de MMP-3 et MMP-13 en présence ou en absence de GIP (10 nM) ; C : expression de MMP-3 et MMP-13 en présence ou en absence de GLP-1 (7-37) (10 nM) ; D : expression de MMP-9 en présence ou en absence de GLP-1 (7-36) amide (10 nM).

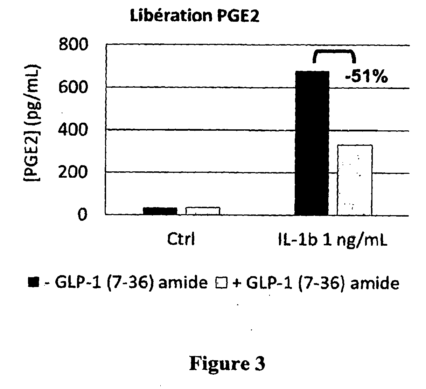

Figure 3 : Effet du peptide GLP-1 (7-36) amide sur l'induction de la libération de prostaglandine E2 par les chondrocytes en réponse à IL-1β (1 ng/mL).

### DESCRIPTION DETAILLEE DE L'INVENTION

Les hormones incrétines GLP-1 (Glucagon-like peptide 1) et GIP (Gastric inhibitory peptide ou glucose-dependent insulinotropic polypeptide) sont des hormones gastro-intestinales libérées par des cellules endocrines de l'épithélium intestinal en réponse à l'absorption de nutriments. Le peptide GLP-1 est produit dans les cellules L de l'iléum ou du colon par clivage protéolytique de la molécule de pré-pro-glucagon sous la forme d'un peptide inactif de 37 acides aminés. Les six résidus N-terminaux sont ensuite clivés pour obtenir les deux formes actives présentes dans le sang : GLP-1 (7-37) et la forme majoritaire GLP-1 (7-36)- amide (Vahl et al.,2003). L'hormone GIP est un peptide de 42 acides aminés sécrété par les cellules K du duodénum. Une fois dans la circulation, ces hormones sont rapidement inactivées par la dipeptidyl-peptidase IV (DPP-IV) (Deacon et al., 2005 ; Orskov et al., 1993).

La sécrétion des hormones incrétines est stimulée par le glucose dont elles potentialisent l'effet sur les cellules β pancréatiques sécrétrices d'insuline. Elles ont donc un effet insulinotrope qui n'est observé qu'en réponse à une augmentation de la glycémie (glycémie post-prandiale) (Kreymann et al., 1987). De nombreuses études ont également révélé qu'elles augmentent la masse des cellules β pancréatiques et la synthèse d'insuline dans ces cellules (Kim et al., 2005 ; Buteau et al., 2008). Le peptide GLP-1 inhibe en outre la sécrétion de glucagon, une hormone hyperglycémiante (Heller et al., 1997).

Contrairement au peptide GIP, l'hormone GLP-1 conserve cet effet insulinotrope chez les patients atteints de diabète de type 2. Elle présente donc un grand intérêt thérapeutique dans le traitement de cette pathologie, d'autant que l'effet insulinotrope est dépendant du glucose et que les risques d'hypoglycémie liée à l'administration de GLP-1, même à des doses élevées, sont ainsi limités. Des analogues de GLP-1 résistant à la DDP-IV, tels que l'exenatide ou le liraglutide, ont ainsi été développés et sont actuellement utilisés dans le traitement du diabète.

Les hormones incrétines agissent également sur l'hypothalamus, le tractus gastrointestinal et le système cardiovasculaire (Zhao et al., 2006). En particulier, elles ralentissent la vidange gastrique et aident à réduire la quantité de nourriture ingérée par le sujet en contribuant à l'apparition d'une sensation de satiété (Gutzwiller et al., 1999). Le peptide GIP a en outre des effets spécifiques sur le tissu adipeux et semble améliorer l'utilisation des lipides absorbés (Yip and Wolfe, 2000). Il pourrait donc être envisagé d'utiliser ces hormones dans le traitement de l'obésité (Neff and Kushner, 2010).

Les inventeurs ont à présent démontré que les incrétines pouvaient avoir un effet thérapeutique dans le cadre d'une toute autre pathologie : l'arthrose. Ils ont en effet montré que la présence de peptide GLP-1 (7-37) (SEQ ID NO: 3), GLP-1 (7-36)- amide (SEQ ID NO: 4) ou GIP (SEQ ID NO: 6) dans les milieux de culture de chondrocytes et d'ostéoblastes permettait d'inhiber la surexpression par ces cellules de plusieurs métalloprotéases (MMP-3, MMP-9 et MMP-13) en réponse à un traitement par la cytokine IL-1β qui est un médiateur majeur de la destruction arthrosique du cartilage. Ils ont également démontré que ces peptides inhibent la libération de la prostaglandine pro-inflammatoire E2 (PGE2), un autre médiateur prodégradatif, par les chondrocytes en réponse à IL-1β.

Les inventeurs ont ainsi mis en évidence l'action inhibitrice directe des hormones incrétines GLP-1 et GIP sur plusieurs facteurs impliqués dans la destruction du cartilage dans le processus arthrosique. L'utilisation de ces hormones, ou leurs analogues, représentent donc une nouvelle stratégie de traitement de l'arthrose. Cette stratégie est d'autant plus avantageuse que plusieurs analogues de GLP-1 ont déjà prouvés leur innocuité et ont été approuvés par les autorités de santé.

La présente invention concerne donc un peptide choisi dans le groupe constitué du peptide GLP-1, du peptide GIP et des analogues de ceux-ci résistant à la dipeptidyl-peptidase IV (DPP-IV), pour une utilisation dans le traitement de l'arthrose.

Dans le présent document, les termes « peptide », « oligopeptide », « polypeptide » ou « protéine » sont utilisés indifféremment et se réfèrent à une chaîne d'acides aminés reliés par des liaisons peptidiques, quel que soit le nombre de résidus d'acide aminé constituant cette chaîne.

Selon un mode de réalisation, le peptide utilisé selon l'invention est choisi dans le groupe constitué du peptide GLP-1 et du peptide GIP. Les peptides GLP-1 ou GIP peuvent être administrés sous une forme mature ou sous la forme d'un précurseur. Le peptide GLP-1 peut ainsi être administré sous la forme d'un précurseur protéique : le pré-pro-glucagon (SEQ ID NO: 1) ou le précurseur GLP-1 (1-37) (SEQ ID NO: 2), ou sous une forme active : le peptide GLP-1 (7-37) (SEQ ID NO: 3) ou le peptide GLP-1 (7-36)- amide (SEQ ID NO: 4). Le peptide GIP peut également être administré sous la forme de son précurseur (SEQ ID NO: 5) ou sous sa forme active GIP (1-42) (SEQ ID NO: 6). Selon un mode de réalisation particulier, le peptide utilisé selon l'invention comprend, ou consiste en, une séquence choisie dans le groupe constitué des séquences SEQ ID NO: 1 à 6. Selon un mode de réalisation préféré, le peptide est choisi parmi les formes actives de GLP-1 ou GIP, c'est-à-dire dans le groupe constitué des peptides de séquence SEQ ID NO: 3, SEQ ID NO: 4 ou SEQ ID NO: 6. De préférence, le peptide est peptide GLP-1 (7-36)-amide (SEQ ID NO: 4).

Après administration, les peptides GLP-1 et GIP sont rapidement inactivés par l'enzyme dipeptidyl-peptidase IV (DPP-IV) qui clive les deux acides aminés N-terminaux. Ainsi, le temps de demi-vie du peptide GLP-1 ou GIP administré par voie intraveineuse est d'environ 2 minutes. Ces peptides ou leurs précurseurs sont donc de préférence administrés de manière continue, par exemple au moyen d'une perfusion sous-cutanée continue. Ainsi, selon un mode de réalisation particulier, le peptide utilisé selon l'invention est un peptide GLP-1 ou GIP, ou un précurseur de ceux-ci, de préférence un peptide choisi dans le groupe constitué du peptide GLP-1 (7-37) (SEQ ID NO: 3), du peptide GLP-1 (7-36)-amide (SEQ ID NO: 4) et du peptide GIP (SEQ ID NO: 6), de manière plus particulièrement préférée le peptide GLP-1 (7-36)-amide (SEQ ID NO: 4), et le peptide est administré au patient à traiter de manière continue, de préférence au moyen d'une perfusion sous-cutanée continue.

Selon un autre mode de réalisation, le peptide est un analogue du peptide GLP-1 ou GIP, de préférence un analogue résistant à la dipeptidyl-peptidase IV (DPP-IV). Ces analogues, aussi appelés incrétino-mimétiques, sont des agonistes du récepteur au GLP-1 ou du récepteur au GIP qui miment l'action des incrétines mais présentent des propriétés améliorées par rapport au peptide GLP-1 ou GIP telles qu'une résistance accrue à la DDP-IV et donc un temps de demi-vie circulante prolongé.

Les modifications des peptides GLP-1 et GIP peuvent être diverses. Par exemples un ou plusieurs acides aminés de configuration L peuvent être remplacés par des acides aminés de configuration D. Le peptide peut subir une modification post-traductionnelle et/ou une modification chimique additionnelle, en particulier une glycosylation, une amidation, une acylation, une acétylation ou une méthylation. Des groupements protecteurs peuvent également être ajoutés aux extrémités C- et/ou N-terminale. Par exemple, le groupement protecteur à l'extrémité N-terminale peut être une acylation ou une acétylation et le groupement protecteur à l'extrémité C-terminale peut être une amidation ou une estérification. Le peptide de l'invention peut également comprendre des liaisons pseudo-peptidiques remplaçant les liaisons peptidiques « classiques » CONH et conférant une résistance accrue aux peptidases, telles que CHOH-CH2, NHCO, CH2-O, CH2CH2, CO-CH2, N-N, CH=CH, CH2NH, et CH2-S. Un ou plusieurs acides aminés peuvent également être remplacés par des acides aminés rares notamment l'hydroxyproline, l'hydroxylysine, l'allohydroxylysine, la 6-N-méthylysine, la N-éthylglycine, la N-méthylglycine, la N-éthylasparagine, l'allo-isoleucine, la N-méthylisoleucine, la N-méthylvaline, la pyroglutamine, l'acide aminobutyrique ; ou des acides aminés synthétiques notamment l'ornithine, la norleucine, la norvaline et la cyclohexyl-alanine. L'invention couvre également l'utilisation des analogues obtenus en soumettant le peptide GLP-1 ou GIP à des substitutions conservatives. Le terme « substitution conservative » tel qu'utilisé dans ce document, se réfère à une substitution d'un résidu d'acide aminé par un autre qui présente des propriétés chimiques ou physiques similaires (taille, charge ou polarité). A titre d'exemple, l'isoleucine, la leucine, l'alanine, la valine, la phénylalanine, la proline et la glycine peuvent être substitués mutuellement de façon conservative, de même que la lysine, l'histidine et l'arginine ou la sérine, la tyrosine et la thréonine ou la cystéine et la méthionine ou l'asparagine, la glutamine et le tryptophane ou l'acide aspartique et l'acide glutamique.

Les analogues des peptides GLP-1 et GIP peuvent présenter un degré d'homologie plus ou moins grand avec ces derniers. A titre d'exemple, le liraglutide, un analogue de GLP-1, présente 97% d'homologie avec le peptide GLP-1 humain alors que l'exendine-4, un autre analogue de GLP-1 obtenu à partir du venin de lézard, ne présente que 53% d'homologie. Cependant, de manière préférée, le peptide est un analogue du peptide GLP-1 ou GIP résistant à la dipeptidyl-peptidase IV (DPP-IV) présentant au moins 50% d'homologie avec le peptide GLP-1 humain, de préférence au moins 60, 70, 80, 90 ou 95% d'homologie avec la SEQ ID NO : 3 ou 4.

De nombreux analogues de GLP-1 et GIP résistant à la DPP-IV ont été décrits et sont issus de différentes stratégies destinées à améliorer leur résistance et augmenter leur temps de demi-vie pour diminuer leur fréquence d'administration.

Des analogues du peptide GLP-1 résistant à la DPP-IV peuvent notamment être obtenus (a) en effectuant des substitutions sélectives d'acides aminés (voir par exemple le brevet américain US 5,545,618), par exemple en substituant le deuxième acide aminé N-terminal, une L-alanine, par une D-alanine ou une sérine, (b) en attachant des substituants lipophiles aux chaînes latérales des résidus d'acides aminés du peptide GLP-1 (voir par exemple le brevet européen EP 0944648), (c) en identifiant des composés insulinotropes et en testant leur capacité d'agonistes du récepteur au GLP-1 humain (par exemple l'exendine 3 et 4 isolés initialement du venin de lézard), (d) en acétylant le peptide GLP-1 ou un de ses analogues, comme cela a été fait pour le liraglutide dans lequel un groupe palmitoyl en C-16 est lié à une lysine d'un peptide GLP-1 modifié, (e) en liant de façon covalente une protéine plasmatique, de préférence l'albumine, à un peptide GLP-1 (par exemple l'albugon (GlaxoSmithKline), le CJC-1131 (ConjuChem), ou le CJC-1134, un analogue de l'exendine-4 lié de façon covalente à une albumine humaine (ConjuChem)), (f) en piégeant les peptides GLP-1 dans des polymères biodégradables, ou (g) en conjuguant le peptide GLP-1 (ou un de ses analogues) avec un polyéthylène glycol (par exemple LY315902, LY2199265 et LY2428757 (Eli Lilly)). Des analogues de GLP-1 ont été décrits dans des nombreux documents brevets tels que US 2011/0281797, WO 2011/134284 ou US 2011/0166321.

Le peptide GIP peut également être modifié pour augmenter sa résistance à la DDP-IV, par exemple en modifiant la tyrosine N-terminale (O'Harte, et al, 1999), en substituant le deuxième acide aminé N-terminal, une L-alanine, par une D-alanine ou une sérine (Hinke, et al, 2002), en mutant l'acide glutamique en position 3 de la séquence SEQ ID NO : 6 (Gault, et al, 2003), ou l'alanine en position 13 de la séquence SEQ ID NO : 6 (Gault, et al, 2003b). Un analogue tronqué de GIP a également été décrit, l'analogue D-Ala²-GIP (1-30) dans lequel la L-alanine en seconde position N-terminale à été substituée par une D-alanine (Widenmaier et al., 2010). Des analogues du peptide GIP présentant une résistance accrue à la DDP-IV ont été décrits dans de nombreux documents brevets tels que par exemple dans les demandes internationales de brevet WO 00/58360, WO 98/24464, WO 03/082898 et WO 2010/016944 ainsi que dans le brevet européen EP 0479210.

Selon un mode de réalisation particulier, le peptide utilisé selon l'invention est un analogue du peptide GLP-1 résistant à la dipeptidyl-peptidase IV, de préférence choisi dans le groupe constitué de l'exenatide (Amylin pharmaceuticals), de l'exendine-4, du liraglutide (Novo Nordisk), de l'albiglutide (ou albugon) (GlaxoSmithKline), le taspoglutide (Ipsen/Roche), le lixisenatide (Sanofi), le LY315902 (Eli Lilly), le dulaglutide (LY2189265) (Eli Lilly), le LY2199265 (Eli Lilly), le LY2428757 (Eli Lilly), le semaglutide NN9535 (Novo Nordisk), le CJC-1131 (ConjuChem), le CJC-1134 (ConjuChem) et le ZP10 (Aventis/ Zealand Pharma). De façon plus particulièrement préférée, le peptide est un analogue choisi dans le groupe constitué de l'exenatide et du liraglutide.

L'invention couvre également l'utilisation des sels pharmaceutiquement acceptables d'un peptide utilisé selon l'invention. Les sels pharmaceutiquement acceptables peuvent être, par exemple, les sels avec des acides minéraux pharmaceutiquement acceptables tels que l'acide chlorhydrique, l'acide bromhydrique, l'acide sulfurique et l'acide phosphorique ; les sels avec des acides organiques pharmaceutiquement acceptables tels que l'acide acétique, l'acide citrique, l'acide maléique, l'acide malique, l'acide succinique, l'acide ascorbique et l'acide tartrique ; les sels avec des bases minérales pharmaceutiquement acceptables tels que les sels de sodium, de potassium, de calcium, de magnésium ou d'ammonium ; ou les sels avec des bases organiques qui ont un azote salifiable, couramment utilisés dans la technique pharmaceutique. Les méthodes de préparation de ces sels sont bien connues de l'homme du métier.

Le peptide selon l'invention peut être obtenu par synthèse chimique classique (en phase solide ou en phase homogène liquide) ou par synthèse enzymatique. Il peut également être obtenu par la méthode consistant à cultiver une cellule hôte comprenant un transgène codant pour le peptide et exprimant ledit peptide, et à extraire ledit peptide à partir de ces cellules hôtes ou du milieu de culture dans lequel le peptide a été sécrété.

Le peptide utilisé selon l'invention peut être utilisé seul ou en combinaison avec un ou plusieurs autres peptides utilisés selon l'invention. Les peptides peuvent être administrés simultanément ou séquentiellement.

Le peptide utilisé selon l'invention peut être utilisé en tant que seul principe actif ou en combinaison avec une ou plusieurs autres substances actives. Le peptide et ladite ou lesdites substances actives peuvent être administrés simultanément ou séquentiellement.

En particulier, le peptide peut être utilisé en combinaison avec un ou plusieurs inhibiteurs de l'enzyme dipeptidyl-peptidase IV. Selon un mode de réalisation, le peptide est utilisé en combinaison avec un ou plusieurs inhibiteurs de l'enzyme dipeptidyl-peptidase IV choisi dans le groupe constitué de la sitagliptine, la saxagliptine, la vildagliptine, l'alogliptine et la linagliptine. Selon un mode particulier de réalisation, le peptide utilisé en combinaison avec un ou plusieurs inhibiteurs de l'enzyme dipeptidyl-peptidase IV est un peptide GLP-1 ou GIP, ou un précurseur de ceux-ci, en particulier un peptide qui comprend, ou consiste en, une séquence choisie dans le groupe constitué des séquences SEQ ID NO: 1 à 6. De préférence, le peptide est choisi dans le groupe constitué du peptide GLP-1 (7-37) (SEQ ID NO: 3), du peptide GLP-1 (7-36)-amide (SEQ ID NO: 4) et du peptide GIP (SEQ ID NO: 6), et de manière plus particulièrement préférée le peptide est le peptide GLP-1 (7-36)-amide (SEQ ID NO: 4).

Le peptide peut également être utilisé en combinaison avec une ou plusieurs substances utilisées dans le traitement de l'arthrose, en particulier des antalgiques tels que le paracétamol ; des anti-inflammatoires non stéroïdiens tels que l'acide acétylsalicylique, l'acétylsalicylate de lysine, le phénylbutazone, le sulindac, le diclofénac potassique ou sodique, l'acéclofénac, l'acide tiaprofénique l'ibuprofène, le kétoprofène, l'alminoprofène, le fénoprofène, le naproxène, le flurbiprofène, l'indométacine, l'acide méfénamique, l'acide niflumique, le ténoxicam, le méloxicam, le piroxicam, et les inhibiteurs sélectifs de la cyclo-oxygénase -2 tels que le célécoxib et l'étoricoxib; les anti-inflammatoires stéroïdiens tels que le bétaméthasone, la dexaméthasone, la prednisolone, la prednisone, le tixocortol ou le triamcinolone; ou des antiarthrosiques à action lente tels que les médicaments comprenant de la chondroïtine, du sulfate de chondroïtine (Structum, Chondrosulf), de la glucosamine ou du sulfate de glucosamine, de la diacéréine (Art 50, Zondar), ou des extraits insaponifiables d'avocat et de soja (Piasclédine).

Le peptide utilisé selon l'invention peut également être administré en association avec des traitements locaux tels que la corticothérapie intra ou péri-articulaire, la visco supplémentation, le lavage articulaire; des traitements chirurgicaux de réaxation ou prosthétiques; ou l'utilisation d'orthèses.

Le peptide utilisé selon l'invention peut également être administré sous la forme d'un acide nucléique codant pour ledit peptide. Tel qu'utilisé dans ce document, on entend par « acide nucléique » toute molécule à base d'ADN ou d'ARN. Il peut s'agir de molécules synthétiques ou semi-synthétiques, recombinantes, éventuellement amplifiées ou clonées dans des vecteurs, modifiées chimiquement, comprenant des bases non-naturelles ou des nucléotides modifiés comprenant par exemple une liaison modifiée, une base purique ou pyrimidique modifiée, ou un sucre modifié. L'acide nucléique peut être sous la forme d'ADN et/ou d'ARN, simple brin ou double brin. Selon un mode de réalisation préférée, l'acide nucléique est une molécule d'ADN isolée, synthétisée par des techniques recombinantes bien connues de l'homme du métier. L'acide nucléique peut être déduit de la séquence du peptide utilisé selon l'invention et l'usage des codons peut être adapté selon la cellule hôte dans laquelle l'acide nucléique doit être transcrit. Ces étapes peuvent être réalisées selon des méthodes bien connues de l'homme du métier et dont certaines sont décrites dans le manuel de référence Sambrook *et al. (Sambrook et al.,* 2001). De préférence, le véhicule utilisé pour administrer l'acide nucléique le protège d'une éventuelle dégradation pouvant nuire à son efficacité. Parmi les véhicules utilisables, on peut notamment citer les polymères cationiques naturels tels que le chitosan ou l'atelocollagène, ou synthétiques tels que le poly(L-lysine), le polyéthylènimine (PEI) ou les dendrimères, qui forment des complexes avec les acides nucléiques; les liposomes ; les liposomes cationiques ; les liposome galactosylés ; les liposomes recouverts d'un ligand leur permettant de cibler un type de cellules tels que les immunoliposomes recouvert d'un anticorps spécifique de la cellule cible (Zheng et al., 2009) ; les liposomes disposés au sein d'une nanoparticule formée par des polymères (Carmona et al., 2009) ou encore des films multicouches de polycations et polyanions.

Tel qu'utilisé dans ce document, le terme "traitement" ou "thérapie" se réfère à tout acte permettant de diminuer, de supprimer ou de retarder les symptômes associés à une pathologie. Il comprend aussi bien un traitement curatif qu'un traitement prophylactique d'une maladie. Un traitement curatif est défini par un traitement aboutissant à une guérison ou un traitement allégeant, améliorant et/ou éliminant, réduisant et/ou stabilisant les symptômes d'une maladie ou la souffrance qu'elle provoque. Un traitement prophylactique comprend aussi bien un traitement aboutissant à la prévention d'une maladie qu'un traitement réduisant et/ou retardant l'incidence d'une maladie ou le risque qu'elle survienne. En particulier, dans le cadre de la présente invention, le terme « traitement » se réfère plus particulièrement à l'inhibition ou au ralentissement de la destruction du cartilage arthrosique observée par le pincement de l'interligne articulaire sur des radiographies standards.

Le peptide utilisé selon l'invention peut être utilisé dans le traitement d'une arthrose primitive (sans cause anatomique ou traumatique) ou secondaire. L'arthrose traitée peut toucher n'importe quelle articulation, notamment les articulations de la hanche (coxarthrose), du genou (gonarthrose), de la cheville, du pied, de la main, du poignet, du coude, de l'épaule ou du rachis, de préférence les articulations de la hanche, du genou, de la main et du rachis.

Le sujet à traiter, ou patient, est un animal, de préférence un mammifère. Selon un mode de réalisation, le sujet à traiter est un animal sélectionné dans le groupe constitué d'un chien, un chat, un cheval, une vache, un mouton, un cochon et un primate non-humain. Selon un mode de réalisation préférée, le sujet à traiter est un humain, de préférence un adulte, et de manière tout particulièrement préférée, un adulte âgé de plus de 50 ans.

Selon un mode particulier de réalisation, le sujet à traiter ne présente pas de diabète de type II. De préférence, ce patient a une glycémie à jeun inférieure à 1,26 g/L.

Selon un autre mode particulier de réalisation, le sujet à traiter ne présente pas d'obésité. L'indice de masse corporelle (ou IMC, masse/(taille)²) permet d'estimer la corpulence d'une personne. Selon la classification de l'OMS, une corpulence moyenne correspond à un IMC de 18,5 à 25, une personne en surpoids a un IMC de 25 à 30 et une personne obèse a un IMC supérieur à 30. De préférence, le sujet à traiter a un indice de masse corporelle inférieur à 30, de manière plus particulièrement préférée, inférieur à 27, et de manière tout particulièrement préférée, inférieur à 25.

Selon encore un autre mode particulier de réalisation, le sujet à traiter a une glycémie à jeun inférieure à 1,26 g/L et un indice de masse corporelle inférieur à 30, de préférence inférieure à 27, et de manière particulièrement préférée inférieur à 25.

Le peptide utilisé selon l'invention est administré au patient sous la forme d'une composition pharmaceutique comprenant au moins un peptide utilisé selon l'invention et un support et/ou excipient pharmaceutiquement acceptable. Le peptide utilisé selon l'invention peut également être administré au patient sous la forme d'une composition pharmaceutique comprenant au moins un acide nucléique codant pour un peptide utilisé selon l'invention et un support et/ou excipient pharmaceutiquement acceptable. La composition peut en outre comprendre une ou plusieurs autre substances actives telles que définies ci-dessus. Elle peut notamment comprendre un ou plusieurs inhibiteurs de l'enzyme dipeptidyl-peptidase IV, de préférence choisis dans le groupe constitué de sitagliptine, la saxagliptine, la vildagliptine, l'alogliptine et la linagliptine, ou d'autres substances telles que des antalgiques, des anti-inflammatoires non stéroïdiens, des anti-inflammatoires stéroïdiens et des antiarthrosiques à action lente.

Les excipients et supports pharmaceutiquement acceptables susceptibles d'être utilisés sont bien connus de l'homme du métier (Remington's Pharmaceutical Sciences, 18th edition, A. R. Gennaro, Ed., Mack Publishing Company [1990]; Pharmaceutical Formulation Development of Peptides and Proteins, S. Frokjaer and L. Hovgaard, Eds., Taylor & Francis [2000]; and Handbook of Pharmaceutical Excipients, 3rd edition, A. Kibbe, Ed., Pharmaceutical Press [2000]). La composition pharmaceutique comprenant le peptide utilisé selon l'invention peut être sous forme de comprimés, capsules, gélules, granulats, suspensions, émulsions, solutions, polymères, nanoparticules, microsphères, suppositoires, lavements, gels, pates, onguents, crèmes, emplâtres, potions, injectables, implants, sprays ou aérosols. De préférence, la composition pharmaceutique comprenant le peptide utilisé selon l'invention est sous la forme d'une composition injectable.

Le peptide utilisé selon l'invention peut être administré par toute voie d'administration connue, incluant notamment les voies systémique (parentérale, intraveineuse...), orale, rectale, topique ou sous-cutanée. Selon un mode de réalisation préféré, le peptide utilisé selon l'invention est administré par voie orale, sous-cutanée ou intraveineuse, de préférence par voie sous-cutanée ou intraveineuse, et de manière tout particulièrement préférée par voie sous-cutanée. Le peptide peut également être administré par injection intra-articulaire, de préférence au niveau de l'articulation arthrosique. Dans ce cas, il peut être administré en combinaison avec d'autres substances à action locale telles que l'acide hyaluronique, ou des substances antalgiques.

Le peptide utilisé selon l'invention est administré au patient à une dose thérapeutiquement efficace. Le terme « dose thérapeutiquement efficace » tel qu'utilisé ici se réfère à la quantité nécessaire pour observer une activité thérapeutique ou préventive sur l'arthrose, en particulier la quantité nécessaire pour observer une inhibition ou un ralentissement de la destruction du cartilage arthrosique. La quantité de peptide à administrer ainsi que la durée du traitement sont évaluées par l'homme du métier selon l'état physiologique du sujet à traiter, la nature de ou des articulations arthrosiques à traiter, le peptide choisi ainsi que la voie d'administration utilisée. Le peptide utilisé selon l'invention peut être administré sous la forme d'une dose unique ou de doses multiples.

Selon un mode de réalisation, le peptide est un peptide GLP-1 ou un peptide GIP, ou un précurseur de ceux-ci, et est administré de façon continue, de préférence au moyen d'une perfusion sous-cutanée continue. Selon un autre mode de réalisation, le peptide est un analogue du peptide GLP-1 ou du peptide GIP résistant à la DDP-IV tel que défini ci-dessus et est administré par injection sous-cutanée une à deux fois par jour. Dans le cas où le peptide est administré sous une forme pharmaceutique à libération prolongée (par exemple une forme injectable à libération prolongée d'exenatide, Le Bydureon™), le peptide peut être administré de manière plus espacée, par exemple une fois par semaine.

Le peptide, de préférence un analogue du peptide GLP-1 ou du peptide GIP résistant à la DDP-IV tel que défini ci-dessus, ou un acide nucléique codant pour ledit peptide, peut également être administré par injection intra-articulaire, en particulier à raison d'une à trois injections par trimestre.

La dose à administrer sera dépendante de la nature du peptide utilisé, de la voie d'administration ainsi que de la fréquence des administrations.

Selon un mode de réalisation, le peptide est administré au patient sous la forme d'une composition pharmaceutique comprenant entre 1 µg et 10 mg de peptide par unité de dose, de préférence par voie orale, sous-cutanée ou intraveineuse.

Selon un mode de réalisation, le peptide est administré au patient par voie sous cutanée sous la forme d'une composition pharmaceutique injectable comprenant entre 5µg et 5 mg de peptide par unité de dose. Selon un autre mode de réalisation, le peptide est administré au patient par voie sous cutanée sous la forme d'une composition pharmaceutique injectable comprenant entre 50 ng et 20µg de peptide par kilo de poids corporel par unité de dose.

La présente invention concerne également l'utilisation d'un peptide choisi dans le groupe constitué du peptide GLP-1, du peptide GIP et des analogues de ceux-ci résistant à la dipeptidyl-peptidase IV (DPP-IV) pour la fabrication d'un médicament destiné au traitement de l'arthrose.

La présente invention concerne en outre une méthode de traitement de l'arthrose chez un patient, ladite méthode comprenant l'administration d'une dose thérapeutiquement efficace d'un peptide choisi dans le groupe constitué du peptide GLP-1, du peptide GIP et des analogues de ceux-ci résistant à la dipeptidyl-peptidase IV (DPP-IV) audit patient.

D'autres caractéristiques et avantages de l'invention apparaîtront mieux à la lecture des exemples suivants donnés à titre illustratif et non limitatif.

### EXEMPLES

### Matériel et méthodes

### Culture primaire de chondrocytes

Les chondrocytes murins ont été obtenus après digestion enzymatique de têtes fémorales et de genoux de souriceaux de 4 à 6 jours. Les cellules ont été amplifiées en monocouche pendant 1 semaine (Gosset et al., 2008).

### Culture primaire d'ostéoblastes

Les ostéoblastes murins ont été obtenus après digestion enzymatique de crânes de souriceaux de 4 à 6 jours. Les cellules ont été amplifiées pendant 3 semaines, en présence d'acide ascorbique et de β-glycérophosphate. Les ostéoblastes en fin de culture forment une membrane tridimensionnelle (Sanchez et al., 2009).

### Traitements des cultures primaires

Les cultures ont été sevrées dans un milieu de culture sans sérum pendant 24 h avant traitement. Les chondrocytes ou les ostéoblastes ont ensuite été traités avec 0,1 ou 1 ng/mL d'IL-1β pendant 24 h, en présence ou en absence de GLP-1 (7-36) amide (10 nM), de GIP (10 nM) ou de GLP-1 (7-37) (10nM) (Bachem).

### Analyse de l'expression génique

Les ARN ont été extraits grâce au RNeasy Mini kit (Qiagen) puis rétro-transcrits grâce au kit Omniscript (Qiagen). Le taux d'expression des gènes d'intérêt ont été quantifiés par PCR en temps réel (Light Cycler LC480), par rapport au gène de référence HPRT, en utilisant les amorces suivantes :
HPRT-antisens : ATT CAA ATC CCT GAA GTA CTC AT (SEQ ID No: 7)
HPRT-sens: AGG ACC TCT CGA AGT GT (SEQ ID No: 8)
GLP1R-antisens: CAG TCG GCA GCC TAG AGA GT (SEQ ID No: 9)
GLP1R- sens: CTG CCC AGC AAC ACC AGT (SEQ ID No: 10)
MMP3- sens: TG AAA ATG AAG GGT CTT CCG G (SEQ ID No: 11)
MMP3- antisens: GCA GAA GCT CCA TAC CAG CA (SEQ ID No: 12)
MMP9- sens: AAC TAC GGT CGC GTC CAC T (SEQ ID No: 13)
MMP9- antisens: CCA CAG CCA ACT ATG ACC AG (SEQ ID No: 14)
MMP13- sens: TGA TGG CAC TGC TGA CAT CAT (SEQ ID No: 15)
MMP13- antisens: TGT AGC CTT TGG AAC TGC TT (SEQ ID No: 16)

### Détection de MMP-13 libérée par Western-blot

La quantité de MMP-13 produite par les cellules a été mesurée par Western-blot (SDS-PAGE et transfert sur membrane de nitrocellulose) à partir des surnageants de culture. La protéine MMP-13 a été détectée avec l'anticorps primaire polyclonal anti-MMP-13 (Santacruz) et un anticorps secondaire couplé à HRP. La révélation a été réalisée grâce au kit Western C (Biorad) et la capture d'image grâce au dispositif Fujifilm Image Reader (Fuji).

### Détection de PGE2 libérée Par EIA

La quantité de PGE2 produite par les cellules a été mesurée grâce au kit EIA prostaglandine E2 kit monoclonal (Cayman) à partir des surnageants de culture.

### Résultats

### Expression du récepteur spécifique à GLP-1

Le récepteur spécifique à GLP-1 (GLP-1R) est exprimé *in vitro* par les chondrocytes et les ostéoblastes primaires murins. Les ARNm codant GLP-1R ont été détectés par RT-PCR dans des lysats cellulaires de chondrocytes et d'ostéoblastes à l'état basal. Ces résultats indiquent que les chondrocytes comme les ostéoblastes expriment ce récepteur à leur surface, et sont donc potentiellement sensibles aux effets de ligands de type GLP-1.

### Inhibition de l'expression des enzymes pro-dégradatives MMP-3, MMP-9 et MMP-13 dans les chondrocytes

L'ajout de GLP-1 (7-36) amide, de GIP ou de GLP-1 (7-37) inhibe la surexpression des enzymes pro-dégradatives MMP-3, MMP-9 et/ou MMP-13 observées dans les chondrocytes en réponse à un traitement IL-1β (Figure 1). Les peptides recombinants seuls n'induisent pas la surexpression des enzymes pro-dégradatives.

### Inhibition de l'expression des enzymes pro-dégradatives MMP-3, MMP-9 et MMP-13 dans les ostéoblastes

L'ajout de GLP-1 (7-36) amide, de GLP-1 (7-37) ou de GIP inhibe la surexpression des enzymes pro-dégradatives MMP-3 et MMP-13 observées dans les ostéoblastes en réponse à un traitement IL-1β (Fig. 2A, B et C). L'ajout de GLP-1 (7-36) amide inhibe en outre la surexpression de MMP-9. (Fig. 2D) Les peptides recombinants seuls n'induisent pas la surexpression des enzymes pro-dégradatives.

### Inhibition de la libération de prostaglandine E2 par les chondrocytes

L'ajout de GLP-1 (7-36) amide inhibe la libération de la prostaglandine E2 (PGE2), un médiateur lipidique pro-inflammatoire, par les chondrocytes observée en réponse à un traitement IL-1β (Figure 3). Ce peptide recombinant seul n'induit pas la libération de PGE2.

### REFERENCES BIBLIOGRAPHIQUES

Blanc et al. Osteoarthritis Cartilage 1999; 7: 308-9.
Buteau J. Diabetes Metab 2008;34(Suppl. 2):S73-7.
Carmona et al., Mol Pharm. 2009 Jan 21
Deacon CF. Regul Pept 2005;128:117-24.
Fosang and Little, 2008, August, Nature Reviews Rheumatology 4, 420-427
Gault, et al, 2003, Biochem. Biophys. Res. Commun., 308:207-213
Gault, et al, 2003b, Cell Biol. International, 27:41-46
Goldring et al. Ann Rheum Dis. 2008; 67 Suppl 3:iii75-82.
Gosset et al. Nat Protoc. 2008;3(8):1253-60
Gutzwiller et al. Gut 1999;44:81-6.
Hardy et al. Arthritis Rheum 2002; 46: 1789-1803.
Hashimoto et al. Arthritis Rheum 1998; 41: 1632-8.
Hinke, et al, 2002, Diabetes, 51:656-661
Heller et al. Diabetes 1997;46:785-91
Kim et al. J Biol Chem 2005;280:22297-307
Kreymann et al. Lancet 1987;2:1300-4.
Krzeski et al., Arthritis Res Ther 2007, 9; R109
Little et al. Matrix Biol 2002;21:271-88.
Miwa et al. Osteoarthritis Cartilage. 2000 Jan;B(1)::17-24.
Nagase et al. J Biol Chem 1999;274:21491-4.
Neff and Kushner, Diabetes Metab Syndr Obes. 2010; 3: 263-273
O'Harte, et al, Diabetes. 1999 Apr;48(4):758-65.
Orskov et al. Diabetes 1993;42:658-61.
Rannou et al. Revue du Rhumatisme 2005; 72 ; 322 - 330
Sambrook J, Russell D (2001) Molecular cloning: a laboratory manual, Third Edition Cold Spring Harbor
Sanchez et al. Osteoarthritis Cartilage. 2009 Apr;17(4):473-81
Sanchez et al. Arthritis Rheum. 2012 Apr;64(4):1193-203.
Sellam and Berenbaum, Nature Reviews Rheumatology 6, 625-635 (November 2010)
Stove et al. Pathobiology 2001;69:333-8.
Vahl et al. J Clin Endocrinol Metab. 2003 Apr;88(4):1772-9
Widenmaier et al. PLoS ONE 2010; 5(3): e9590
Yip and Wolfe. Life Sci 2000;66:91-103.
Zheng et al., Blood. 2009 Mar 19;113(12):2646-54Zhao et al. J Pharmacol Exp Ther 2006;317:1106-13.

## Revendications

1. Peptide choisi dans le groupe constitué du peptide GLP-1, du peptide GIP et des analogues de ceux-ci résistant à la dipeptidyl-peptidase IV (DPP-IV), pour une utilisation dans le traitement de l'arthrose.

2. Peptide pour utilisation selon la revendication 1, dans lequel le peptide comprend une séquence choisie dans le groupe constitué des séquences SEQ ID NO: 1 à 6.

3. Peptide pour utilisation selon la revendication 1 ou 2, dans lequel le peptide est choisi dans le groupe constitué du peptide GLP-1 (7-36)- amide (SEQ ID NO: 4) et du peptide GLP-1 (7-37) (SEQ ID NO: 3).

4. Peptide pour utilisation selon la revendication 1 ou 2, dans lequel le peptide est le peptide GIP (1-42) (SEQ ID NO: 6).

5. Peptide pour utilisation selon la revendication 1, dans lequel le peptide est un analogue du peptide GLP-1 ou GIP résistant à la DPP-IV.

6. Peptide pour utilisation selon la revendication 1, dans lequel le peptide est un analogue du peptide GLP-1 résistant à la DPP-IV, de préférence un peptide choisi dans le groupe constitué de l'exenatide, le liraglutide, l'exendine-4, l'albiglutide, le taspoglutide, le lixisenatide, le dulaglutide (LY2189265), le LY315902, le LY2199265, le LY2428757, le semaglutide (NN9535), le CJC-1131, le CJC-1134 et le ZP10, et de manière tout particulièrement préférée un peptide choisi dans le groupe constitué de l'exenatide et du liraglutide.

7. Peptide pour utilisation selon l'une quelconque des revendications 1 à 6 pour une utilisation dans le traitement de l'arthrose en combinaison avec une ou plusieurs autres substances actives.

8. Peptide pour utilisation selon la revendication 7, dans lequel le peptide et ladite ou lesdites autres substances actives sont administrés simultanément ou séquentiellement.

9. Peptide pour utilisation selon l'une quelconque des revendications 7 à 8, dans lequel ladite ou lesdites autres substances actives sont (i) des inhibiteurs de l'enzyme dipeptidyl-peptidase IV, de préférence choisis dans le groupe constitué de sitagliptine, la saxagliptine, la vildagliptine, l'alogliptine et la linagliptine, ou (ii) choisies dans le groupe constitué des antalgiques, des anti-inflammatoires non stéroïdiens, des anti-inflammatoires stéroïdiens et des antiarthrosiques à action lente.

10. Peptide pour utilisation selon l'une quelconque des revendications 1 à 9, dans lequel le peptide est administré en association avec des traitements locaux de l'arthrose.

11. Peptide pour utilisation selon l'une quelconque des revendications 1 à 10, dans lequel le peptide est administré sous la forme d'un acide nucléique codant pour ledit peptide.

12. Peptide pour utilisation selon l'une quelconque des revendications 1 à 11, destiné à être administré par voie orale, sous-cutanée, intraveineuse ou intra-articulaire.

13. Composition pharmaceutique comprenant (i) un ou plusieurs peptides tels que définis dans l'une quelconque des revendications 1 à 6 ou (ii) un ou plusieurs acides nucléiques codant pour un ou plusieurs peptides tels que définis dans l'une quelconque des revendications 1 à 6, et un support et/ou excipient pharmaceutiquement acceptable, pour une utilisation dans le traitement de l'arthrose.

14. Composition pharmaceutique pour utilisation selon la revendication 13, ladite composition comprenant en outre une ou plusieurs autres substances actives, de préférence (i) un ou plusieurs inhibiteurs de l'enzyme dipeptidyl-peptidase IV, de préférence choisis dans le groupe constitué de sitagliptine, la saxagliptine, la vildagliptine, l'alogliptine et la linagliptine, ou (ii) une ou plusieurs autres substances actives choisies dans le groupe constitué des antalgiques, des anti-inflammatoires non stéroïdiens, des anti-inflammatoires stéroïdiens et des antiarthrosiques à action lente.

15. Composition pharmaceutique pour utilisation l'une quelconque des revendications 13 à 14, ladite composition étant formulée sous la forme d'une composition ingérable ou injectable, de préférence sous la forme d'une composition destinée à être administré par voie orale, sous-cutanée, intraveineuse ou intra-articulaire.

## Patentansprüche

1. Peptid, ausgewählt aus der Gruppe, bestehend aus Peptid GLP-1, Peptid GIP und Analoga davon, die gegen die Dipeptidyl-Peptidase IV (DPP-IV) resistent sind, für eine Verwendung in der Behandlung der Arthrose.

2. Peptid zur Verwendung gemäß Anspruch 1, wobei das Peptid eine Sequenz umfasst, ausgewählt aus der Gruppe, bestehend aus den Sequenzen SEQ ID NR: 1 bis 6.

3. Peptid zur Verwendung gemäß Anspruch 1 oder 2, wobei das Peptid aus der Gruppe ausgewählt ist, bestehend aus Amidpeptid GLP-1 (7-36) (SEQ ID NR: 4) und Peptid GLP-1 (7-37) (SEQ ID NR: 3).

4. Peptid zur Verwendung gemäß Anspruch 1 oder 2, wobei das Peptid das Peptid GIP (1-42) (SEQ ID NR: 6) ist.

5. Peptid zur Verwendung gemäß Anspruch 1, wobei das Peptid ein Analogon des Peptids GLP-1 oder GIP ist, das gegen DPP-IV resistent ist.

6. Peptid zur Verwendung gemäß Anspruch 1, wobei das Peptid ein Analogon des Peptids GLP-1, das gegen DPP-IV resistent ist, vorzugsweise ein Peptid ist, ausgewählt aus der Gruppe, bestehend aus Exenatid, Liraglutid, Exendin-4, Albiglutid, Taspoglutid, Lixisenatid, Dulaglutid (LY2189265), LY315902, LY2199265, LY2428757, Semaglutid (NN9535), CJC-1131, CJC-1134 und ZP10 und ganz besonders bevorzugt ein Peptid ist, ausgewählt aus der Gruppe, bestehend aus Exenatid und Liraglutid.

7. Peptid zur Verwendung gemäß einem der Ansprüche 1 bis 6 für eine Verwendung in der Behandlung der Arthrose in Kombination mit einem oder mehreren weiteren Wirkstoffen.

8. Peptid zur Verwendung gemäß Anspruch 7, wobei das Peptid und besagter oder besagte weitere Wirkstoffe gleichzeitig oder aufeinanderfolgend verabreicht werden.

9. Peptid zur Verwendung gemäß einem der Ansprüche 7 bis 8, wobei besagter oder besagte weitere Wirkstoffe (i) Hemmer des Enzyms Dipeptidyl-Peptidase IV sind, vorzugsweise ausgewählt aus der Gruppe, bestehend aus Sitagliptin, Saxagliptin, Vildagliptin, Alogliptin und Linagliptin, oder (ii) aus der Gruppe ausgewählt sind, bestehend aus Analgetika, nicht-steroidalen Entzündungshemmern, steroidalen Entzündungshemmern und langsam wirkenden Antiarthrosemitteln.

10. Peptid zur Verwendung gemäß einem der Ansprüche 1 bis 9, wobei das Peptid kombiniert mit lokalen Arthrose-Behandlungen verabreicht wird.

11. Peptid zur Verwendung gemäß einem der Ansprüche 1 bis 10, wobei das Peptid in Form einer Nukleinsäure, die für besagtes Peptid codiert, verabreicht wird.

12. Peptid zur Verwendung gemäß einem der Ansprüche 1 bis 11, das oral, subkutan, intravenös oder intraartikulär verabreicht werden soll.

13. Pharmazeutische Zusammensetzung, umfassend (i) ein oder mehrere Peptide, wie in einem der Ansprüche 1 bis 6 definiert, oder (ii) eine oder mehrere Nukleinsäuren, die für ein oder mehrere wie in einem der Ansprüche 1 bis 6 definierte Peptide codieren, und einen pharmazeutisch verträglichen Träger und/oder Exzipienten für eine Verwendung in der Behandlung der Arthrose.

14. Pharmazeutische Zusammensetzung zur Verwendung gemäß Anspruch 13, wobei besagte Zusammensetzung außerdem umfasst einen oder mehrere weitere Wirkstoffe, vorzugsweise (i) einen oder mehrere Hemmer des Enzyms Dipeptidyl-Peptidase IV, vorzugsweise ausgewählt aus der Gruppe, bestehend aus Sitagliptin, Saxagliptin, Vildagliptin, Alogliptin und Linagliptin, oder (ii) einen oder mehrere weitere Wirkstoffe, ausgewählt aus der Gruppe, bestehend aus Analgetika, nicht-steroidalen Entzündungshemmern, steroidalen Entzündungshemmern und langsam wirkenden Antiarthrosemitteln.

15. Pharmazeutische Zusammensetzung zur Verwendung gemäß einem der Ansprüche 13 bis 14, wobei besagte Zusammensetzung in Form einer einnehmbaren oder injizierbaren Zusammensetzung formuliert ist, vorzugsweise in Form einer Zusammensetzung, die oral, subkutan, intravenös oder intraartikulär verabreicht werden soll.

## Claims

1. A peptide selected from the group consisting of the GLP-1 peptide, the GIP peptide and analogues thereof resistant to dipeptidyl peptidase IV (DPP-IV), for use in the treatment of osteoarthritis.

2. The peptide for use according to claim 1, wherein the peptide comprises a sequence selected from the group consisting of the sequences SEQ ID NO: 1 to 6.

3. The peptide for use according to claim 1 or 2, wherein the peptide is selected from the group consisting of the GLP-1 (7-36) amide peptide (SEQ ID NO: 4) and the GLP-1 (7-37) peptide (SEQ ID NO: 3).

4. The peptide for use according to claim 1 or 2, wherein the peptide is the GIP (1-42) peptide (SEQ ID NO: 6).

5. The peptide for use according to claim 1, wherein the peptide is an analogue of the GLP-1 or GIP peptide, resistant to DPP-IV.

6. The peptide for use according to claim 1, wherein the peptide is an analogue of the GLP-1 peptide resistant to DPP-IV, preferably a peptide selected from the group consisting of exenatide, liraglutide, exendin-4, albiglutide, taspoglutide, lixisenatide, dulaglutide (LY2189265), LY315902, LY2199265, LY2428757, semaglutide (NN9535), CJC-1131, CJC-1134 and ZP10, and most preferably a peptide selected from the group consisting of exenatide and liraglutide.

7. The peptide for use according to any one of claims 1 to 6, for use in the treatment of osteoarthritis in combination with one or more other active substances.

8. The peptide for use according to claim 7, wherein the peptide and said other active substance(s) are administered simultaneously or sequentially.

9. The peptide for use according to any one of claims 7 to 8, wherein said other active substance(s) is (are) (i) inhibitors of the dipeptidyl peptidase IV enzyme, preferably selected from the group consisting of sitagliptin, saxagliptin, vildagliptin, alogliptin and linagliptin, or (ii) selected from the group consisting of analgesics, non-steroidal anti-inflammatories, steroidal anti-inflammatories and slow-acting anti-arthritic agents.

10. The peptide for use according to any one of claims 1 to 9, wherein the peptide is administered in combination with local treatments for osteoarthritis.

11. The peptide for use according to any one of claims 1 to 10, wherein the peptide is administered in the form of a nucleic acid encoding said peptide.

12. The peptide for use according to any one of claims 1 to 11, intended to be administered orally, subcutaneously, intravenously or intra-articularly.

13. A pharmaceutical composition comprising (i) one or more peptides as defined in any one of claims 1 to 6 or (ii) one or more nucleic acids encoding one or more peptides as defined in any one of claims 1 to 6, and a pharmaceutically acceptable support and/or excipient, for use in the treatment of osteoarthritis.

14. The pharmaceutical composition for use according to claim 13, said composition also comprising one or more other active substances, preferably (i) one or more inhibitors of the dipeptidyl peptidase IV enzyme, preferably selected from the group consisting of sitagliptin, saxagliptin, vildagliptin, alogliptin and linagliptin, or (ii) one or more other active substances selected from the group consisting of analgesics, non-steroidal anti-inflammatories, steroidal anti-inflammatories and slow-acting anti-arthritic agents.

15. The pharmaceutical composition for use according to any one of claims 13 to 14, said composition being formulated in the form of an ingestible or injectable composition, preferably in the form of a composition intended to be administered orally, subcutaneously, intravenously or intra-articularly.
